# EUROPEAN PATENT SPECIFICATION

(11) **EP 2 575 691 B1**
(45) Date of publication and mention of the grant of the patent: **30.12.2015**
(21) Application number: 11787040.2
(22) Date of filing: 27.05.2011
(51) Int. Cl.: A61F 2/46, A61F 2/28, A61F 2/44, A61F 2/30

(54) **SUPPORT DEVICE**
STÜTZVORRICHTUNG
DISPOSITIF DE SUPPORT

(30) Priority: 27.05.2010 US 349151 P
(43) Date of publication of application: 10.04.2013
(73) Proprietor: Flexmedex, LLC, Perkasie, PA 18944 (US)
(72) Inventor: GREENHALGH, E., Skott, Lower Gwynedd PA 19002 (US); ROMANO, John-Paul, Chalfont PA 18914 (US)
(74) Representative: Thoma, Michael
(86) International application number: PCT/US2011/000974
(87) International publication number: WO 2011/149557

(56) References cited:
- WO-A2-2006/072941
- WO-A2-2008/016598
- FR-A1- 2 900 814
- US-A1- 2006 036 241
- US-A1- 2008 312 743
- US-A1- 2009 054 991
- US-A1- 2009 182 431

## Description

### BACKGROUND OF THE INVENTION

### 1. Field of the Invention

A device, such as a flexible spinal fusion cage, which can articulate (bend) in such a way that it will be able to be implanted from a lateral approach into L4-L5 and L5-S1 is disclosed.

### 2. Description of the Related Art

Typical lateral approach fusion implants (e.g., Discover XLIF, by NuVasive, Inc., San Diego, CA; and the Direct Lateral Interbody Fusion (DLIF) by Medtronic, Inc., Minneapolis, MN) are not able to implant their fusion cages for two reasons.

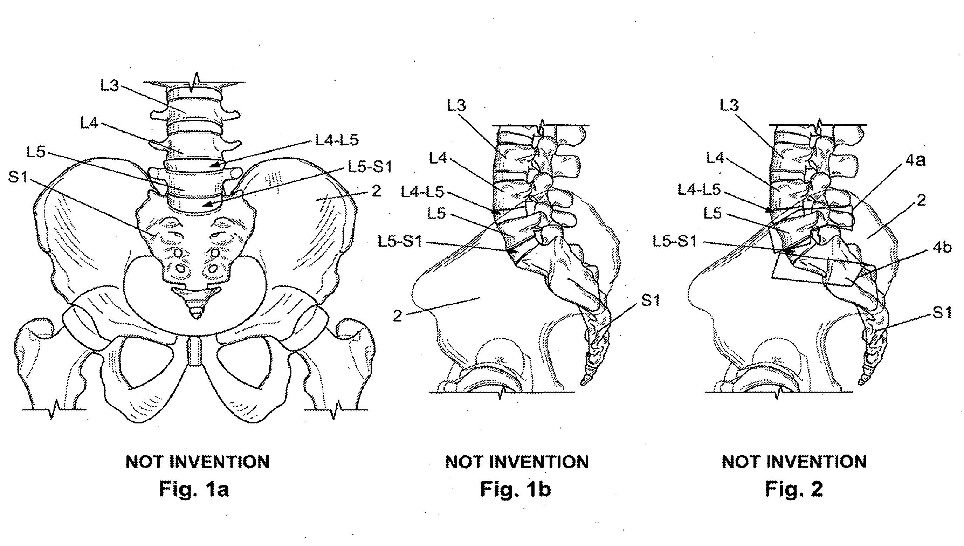

First, boney obstacles can impair access. Figures 1a and 1b illustrate the pelvis and lower spine including the Ilium 2, sacrum S1, and lower lumbar vertebrae L3, L4 and L5. Figures 1a and 1b show the challenge of gaining lateral access to the L4-L5 and the L5-S1 intervertebral spaces. The position of the Ilium 2 obstructs the direct lateral access pathway.

Figure 2 illustrates windows 4a and 4b or channels which some doctors create during implantation. The windows 4a and 4b are created through the Ilium to gain direct line of site access to the L4-L5 and L5-S1 intervertebral spaces, respectively. This is a highly invasive approach, creates significant tissue damage, particularly to the Ilium and surrounding soft tissue, and requires significant surgical skill.

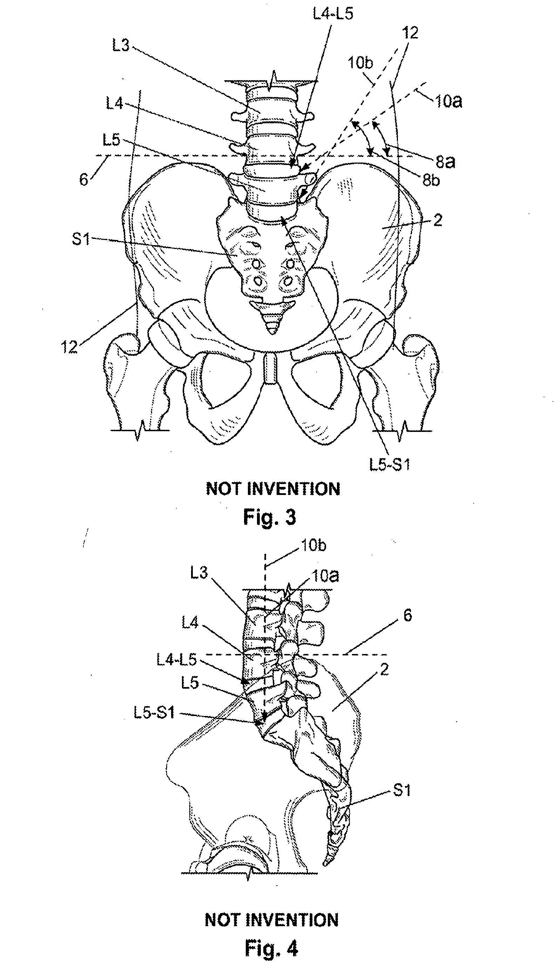

Second, the steep approach angle (8a for the L4-L5 intervertebral space and 8b for the L5-S1 intervertebral space), as measured from a transverse plane along the approach path (10a for the L4-L5 intervertebral space and 10b for the L5-S1 intervertebral space) of a tissue retractor relative to the location of the fusion site, can cause problems, as illustrated in Figures 3 and 4. The approach paths 10a and 10b pass through the skin surface 12. The tissue retractor used in lateral fusion surgery provides line of site access to the disk space requiring a fusion cage insertion. The tissue retractor holds tissue out of the way of the procedure. The tissue retractor is also used to create a working channel to pass tools through, protect neural tissue, and anchor to the superior and inferior vertebral bodies relative the disk space requiring fusion. The volume within the pelvis and inferior to the dashed demarcation line 6 along a transverse plane is very hard if not impossible to reach with a direct lateral approach due to the Ilium. Even if the retractors are tilted as shown by the demarcation line 6, the ability to insert an implant that is the length of the end plates of the L4 or L5 vertebral bodies would be very difficult due to obstruction of the Ilium among other factors.

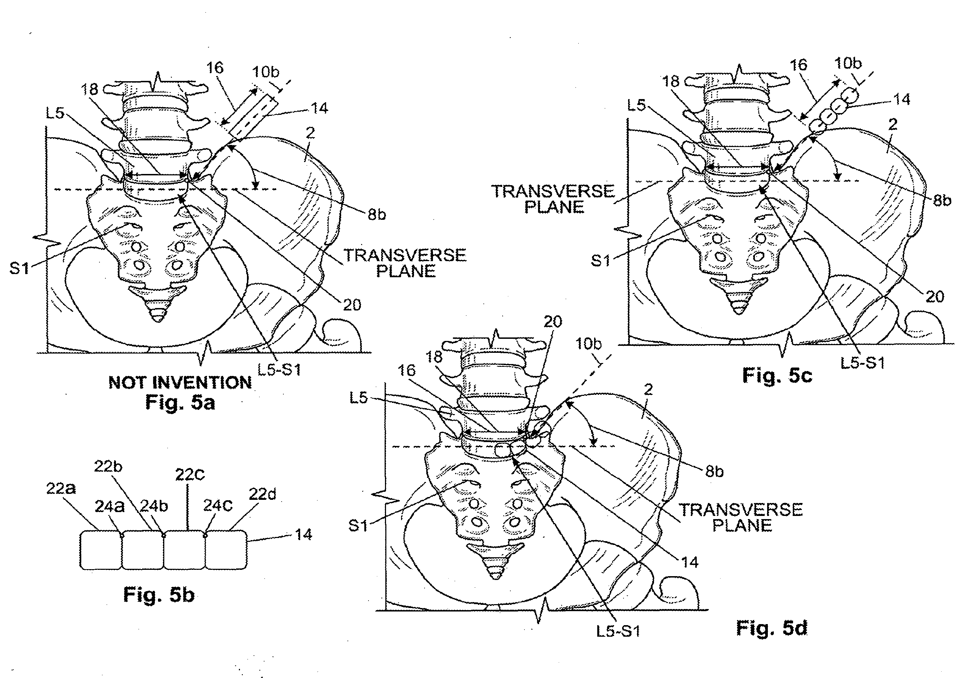

Furthermore, with the retractor positioned along the approach path 10a or 10b plane and angled direction, the angle formed between the retractor and the vertebral body end plates would make inserting a monolithic, inflexible fusion cage 14 or implant into the L5-S1 intervertebral space difficult if not virtually impossible due to obstruction of the surrounding hard and soft tissue, as illustrated by Figure 5a. A typical lateral fusion cage or implant width 16 is the width of the end plate 18 along the adjacent disk. The implant 14 can not turn the corner at the pivot point 20 at the lateral and/or anterior edge of the L5-S1 intervertebral space.

A biological implant device according to the preamble of claim 1 is known from WO 2008/016598 A2 which discloses a spinal implant comprising a cage having flexible joints allowing the cage to be deformed for insertion into a patient.

### SUMMARY OF THE INVENTION

The present invention provides for a biological implant device as defined by claim 1. Preferred embodiments of the invention are laid down in the dependent claims.

Support or fixation devices and methods for access, controlling (e.g., steering or rotating, and driving or translating) implants, and modifying the configuration of implants are disclosed. The device can be an implantable fixation device, such as a flexible and/or articulatable fusion cage. The device can articulate and/or bend so the device can make the turn around the L5-S1 intervertebral space. The implant can flex and/or articulate. For example, the implant can have hinges and/or be flexible (e.g., have significantly elastic structural components).

Articulation tools are disclosed that can be used to implant the device. The articulation tools can articulate the device and/or allow the device to articulate. For example, the connection between the articulation tool and the implant can bend, flex, steer, or combinations thereof. The articulation tools can be used to debride or clear out the disk space.

An oblique curved access tool or device can be used. The device can be delivered to the intervertebral space along an oblique approach path, not perpendicular to the spine. The oblique approach can provide an access path from lateral skin to the L5-S1 disk space, and can curve tangent to the Ilium. A large working channel through the soft tissue can be created. The oblique access tool can move soft tissue out of the way to create the working channel. The oblique approach can reduce the access-tool-to-disk-space approach angle.

### BRIEF DESCRIPTION OF THE DRAWINGS

Figures 1a and 1b are anterior and lateral views, respectively, of the lower lumbar and sacral spine and pelvis with the Ilium shown in phantom lines in Figure 1b.
Figure 2 is a lateral view of the lower lumbar spine with windows cut through the Ilium.
Figures 3 and 4 are anterior and lateral views, respectively, of the lower spine and pelvis along with approach paths into the intervertebral spaces.
Figure 5a is an anterior close-up view of the lower spine and pelvis with an approach of a monolithic implant.
Figure 5b illustrates a variation of the implantable device.
Figures 5c and 5d illustrate a variation of a method of delivering the device of Figure 5b into the L5-S1 space.
Figures 6 through 8 are anterior, perspective and lateral views, respectively, of a variation of the approach path for delivering the implant into the intervertebral space.
Figures 9a through 9d illustrate variations of the device in various configurations. An x-axis, y-axis and z-axis are also shown for orientation with the x-axis disposed along the longitudinal axis of the device.
Figures 10a and 10b illustrate various configurations of a variation of the device in a steering tube with the tube shown as see-through for illustrative purposes.
Figures 10c through 10e illustrate various configurations of a variation of the device on steering rails attached to the lateral outside of the device.
Figures 11a through 11c illustrate various configurations of a variation of the device on a steering rail attached to the inside of the device.
Figures 12a through 12f are cross-sections of various steering rails, or along the length of the same steering rail.
Figure 13 illustrates a method for deploying the device into the L5-S1 intervertebral space.
Figures 14a and 14b illustrate various configurations of a variation of the device in a steering slide.
Figures 15a and 15b are top and side views of a variation of the device with parallel hinges.
Figures 16 is a top views of a variation of the device with non-parallel hinges.
Figures 17a through 17f are side views of variations of the device.
Figures 18 and 19 are perspective views showing the orientation of variations of living hinges within devices.
Figures 20a through 20c are perspective, top and front views, respectively, of a variation of the device in a straight or flat configuration.
Figures 21a through 21c are perspective, top and front views, respectively, of the device of Figures 20a through 20c in an articulated configuration.
Figures 22a through 22c are perspective, top and front views, respectively, of a variation of the device in a straight or flat configuration.
Figures 23a through 23c are perspective, top and front views, respectively, of the device of Figures 22a through 22c in an articulated configuration.

### DETAILED DESCRIPTION

Support or fixation devices and methods for access, controlling (steering) implants, and modifying implants are disclosed. The device can be an implantable fixation device, such as a flexible fusion cage. The device can be delivered into an intervertebral space, for example, to provide structural support between the adjacent vertebrae. The device can fuse the vertebra adjacent to the specific intervertebral space. A discectomy can be performed at the target implant site before or during delivery of the implant.

Figure 5b illustrates that the implantable device 14 can have first, second, third, and fourth segments 22a through 22d. Each of the segments 22a, 22b, 22c, and 22d can be attached to the adjacent segment at a flex point or articulatable hinge 24a, 24b, and 24c, respectively. The device 14 can articulate and/or bend at the hinges 24.

Figures 5c and 5d illustrate that the device 14 can be delivered into the L5-S1 intervertebral space. The device 14 can make the turn around the L5-S1 intervertebral space, such as at the pivot point 20, by articulating or flexing.

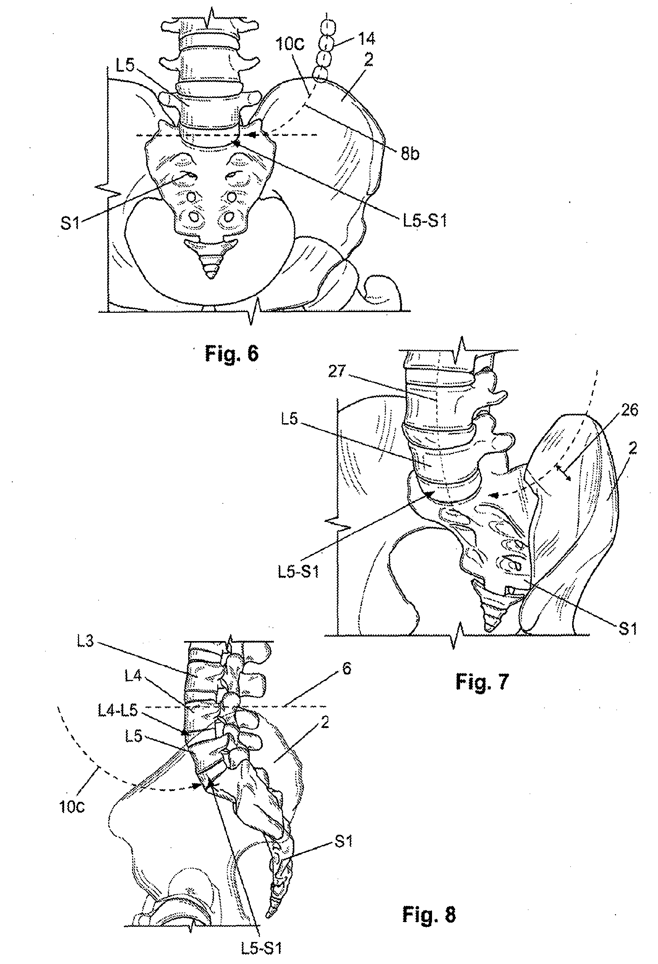

Figures 6 through 8 shows illustrate a curved implant pathway or approach path 10c. An articulation tool can be used to push (e.g., impact), pull, control or combinations thereof, the implant 14. The implant 14 can articulate and/or flex during delivery. The implant can have single or multiple hinges, a flexible shaft, laser slots (e.g., in a tube to act as hinges) or combinations thereof.

The approach path 10c can be tangential to the medial surface of the Ilium along a portion of the length of the approach path 10c. A portion of the length of the approach path 10c can be linear and a portion of the length of the approach path 10c can be curved. The entire approach path 10c can be linear or curved. A portion of the length of the approach path 10c can track (i.e., follow the same shape of) the medial surface of the Ilium. The approach path 10c can contact the medial surface of the Ilium 2. The approach path 10c can be non-perpendicular or perpendicular to the longitudinal axis 27 of the spine where the approach path 10c enters the intervertebral space L4-L5 or L5-S1.

The approach-Ilium gap 26 can be measured between the approach path 10c and the closest medial surface of the Ilium 2. The approach-Ilium gap 26 can be perpendicular to the approach path 10c and the Ilium 2, for example when the approach path 10c is tracking the medial surface of the Ilium 2. The approach-Ilium gap 26 can be from about 0 mm to about 15 mm along the length of the approach path 10c where the approach path is tracking the medial surface of the Ilium 2, more narrowly from about 0 mm to about 10 mm, yet more narrowly from about 2 mm to about 8 mm.

The approach path 10c can be curved in all three dimensions (e.g., in the transverse plane, sagittal plane and coronal plane), or any combination thereof and straight in the remaining dimensions.

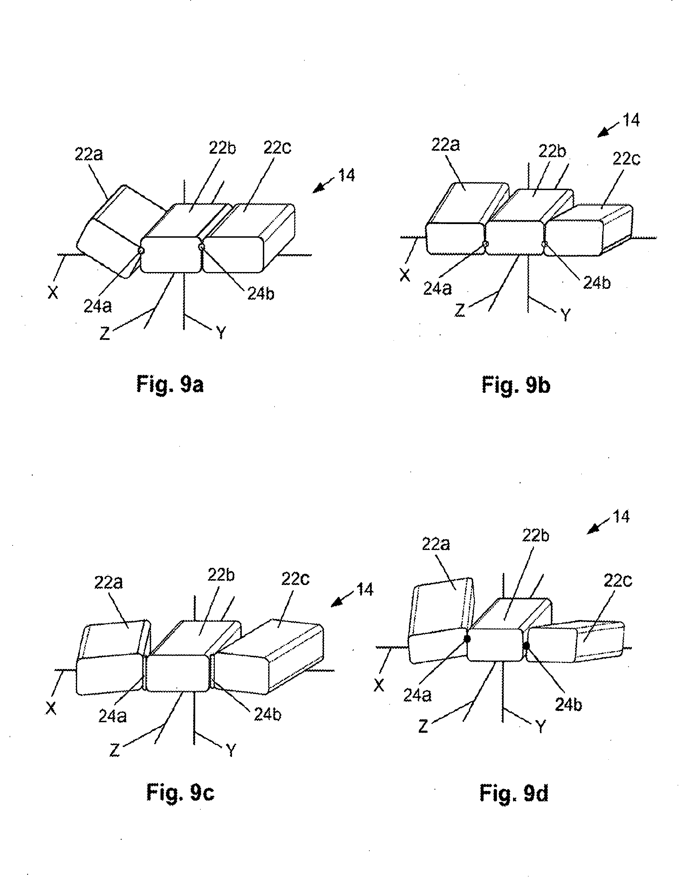

Figure 9a through 9d illustrate that variations of hinges 24a and 24b between the segments 22a, 22b and 22c can allow the implant 14 to articulate. The implant 14 can have controlled angulation or articulation (i.e., with discrete, defined built-in stopping points or stops) or free angulation or articulation (i.e., with no stops).

Figure 9a illustrates that the hinges 24a and 24b can be oriented in parallel with the z-axis. The hinges can have a single degree of rotational freedom. The segments 24, 24b and 24c can articulate by rotating about the z-axis with respect to each other. The hinges 24a and 24b can be near the top (as shown), near the bottom, in the middle with respect to the y-axis, or combinations thereof of the device 14.

Figure 9b illustrates that the hinges 24a and 24b can be oriented in parallel with the x-axis. The segments 24, 24b and 24c can articulate by rotating about the x-axis with respect to each other. The hinges 24a and 24b can be near the front (as shown), near the rear, in the middle with respect to the z-axis, or combinations thereof of the device 14.

Figure 9c illustrates that the hinges 24a and 24b can be oriented in parallel with the y-axis. The segments 24, 24b and 24c can articulate by rotating about the y-axis with respect to each other. The hinges 24a and 24b can be near the front (as shown), near the rear, in the middle with respect to the z-axis, or combinations thereof of the device 14.

Figure 9d illustrates that the hinges 24a and 24b can be ball-in-socket hinges allowing three rotational degrees of freedom, or a combination of the three hinges described in Figures 9a through 9c, allowing two or three degrees of freedom. The segments 24, 24b and 24c can articulate by rotating about the x-axis, and/or y-axis, and/or z-axis with respect to each other. The hinges 24a and 24b can be near the front (as shown), near the rear, in the middle with respect to the z-axis, near the top, near the bottom, in the middle with respect to the y-axis (as shown), or combinations thereof of the device 14.

The first hinge 24a can be located in a different location and/or with a different than the second hinge 24b. For example, the first hinge 24a can be oriented in parallel with the z-axis, allow rotation about the z-axis and be located near the top of the device 14, and the second hinge 24b can be oriented in parallel with the x-axis, allow rotation about the x-axis, and be located near the middle of the device 14 with respect to the z-axis.

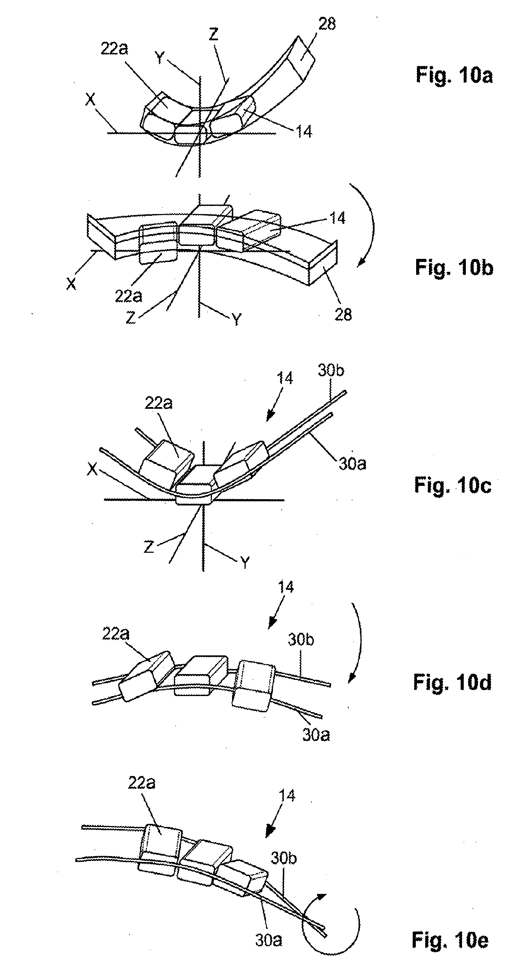

Figures 10a and 10b illustrate that the device 14 can have an outer steering sheath or tube 28. The device 14 can be fixed to the steering tube 28 or can slide along the steering tube 28. The steering tube 28 can be articulatable and/or flexible, as shown by the arrow in Figure 10b and the various configurations of the tube 28 between Figures 10a and 10b. The articulation or flexion of the steering tube 28 can be controlled, for example by delivering controlled tension to tensile control wires in the walls of the steering tube 28.

The steering tube 28 can be positioned at the target deployment site. For example, the steering tube 28 can be placed in the intervertebral space and can remain in the intervertebral space post-surgery, or the steering tube 28 can be removed from the intervertebral space and the device 14 can be deployed from the tube 28 and the device 14 can be left in the intervertebral space.

Also for example, the distal end of the steering tube 28 can be positioned at the entrance to the intervertebral space and/or rested on the inferior and/or superior vertebral body end plate adjacent to the target intervertebral space. The device 14 can then be pushed (e.g., by a plunger) out of the steering tube and into the intervertebral space. The steering tube 28 does not have to, but can, enter the intervertebral space.

Figures 10c through 10d illustrate that the device 14 can have one or more exterior steering rails, tracks or wires 30a and 30b, such as guidewires. The rails 30a and 30b can slidably or fixedly and releasably engage the external surface of the segments 22 of the device 14. For example, the rails can pass through slots, guides, collars, cuffs or combinations thereof on the exterior of the segments 22. The slots, guides, collars, cuffs or combinations thereof, and/or the rails 30a and 30b can be coated or covered with a low-friction (e.g., PTFE) or high-friction (e.g., knurled or toothed surface texturing) material or surface treatment or texture, including any of the materials listed herein. The steering rails 30a and 30b can be steered or manipulated by applying a tensile force to tensile cables within the rails, as shown by the arrows in Figures 10d and 10e, and the flexing from Figure 10c to 10d. The rails 30a and 30b can be pre-formed to a specific shape and can be substituted for other rails 30a and 30b that can be pre-formed to a different shape to change the direction of delivery.

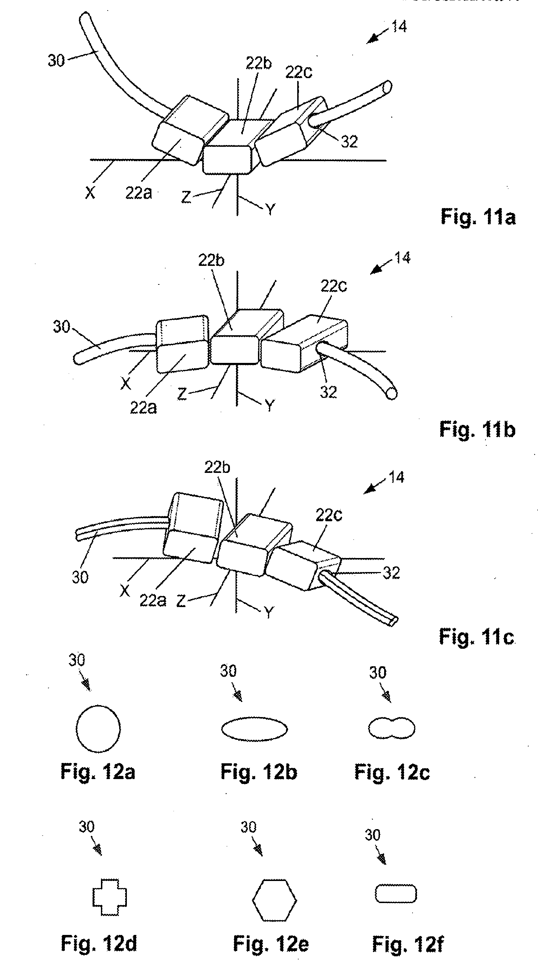

Figures 11a through 11c illustrates that the device 14 can have one or more interior steering rails, guide, tracks or wires 30, such as guidewires. The rails 30 can be positioned through the center or interior of one or more segments 22 of the device 14. The rail 20 can slidably or fixedly and releasably engage an internal surface, such as through a longitudinal guide port or channel 32, of the segments 22 of the device 14. For example, ports or channels can extend longitudinally through the segments 22 of the device 14. The channels, and/or the rail 30 can be coated, covered or collared, such as with a low-friction (e.g., PTFE) or high-friction (e.g., knurled or toothed surface texturing) material or surface treatement or texture, including any of the materials listed herein. The steering rail 30 can be steered or manipulated by applying a tensile force to tensile cables within the rail 30, as shown by the flexing from Figure 11a to 11c. The rail 30 can be pre-formed to a specific shape and can be substituted for one or more other rails 30 that can be pre-formed to a different shape to change the direction of delivery.

The distal ends of the internal and/or external steering rail or rails 30 can be positioned at the target deployment site. For example, the steering rails 30 can be placed in the intervertebral space and can remain in the intervertebral space post-surgery, or the steering rails 30 can be removed from the intervertebral space and the device 14 can be deployed from the rails 30 and the device 14 can be left in the intervertebral space.

Also for example, the distal end of the steering rails 30 can be positioned at the entrance to the intervertebral space and/or rested on the inferior and/or superior vertebral body end plate adjacent to the target intervertebral space. The device 14 can then be pushed (e.g., by a plunger) out of the steering rails 30 and into the intervertebral space. The steering rails 30 do not have to, but can, enter the intervertebral space.

Figures 12a through 12f illustrate cross-sections of various rails 30, or at various lengths along the same rail 30. Figure 12a illustrates that the cross-section of the steering rail 30 can be circular. Figure 12b illustrates that the cross-section of the steering rail 30 can be oval. Figure 12c illustrates that the cross-section of the steering rail 30 can be multi-ovular (i.e., having a union of two or more ovals with the same major axis). Figure 12d illustrates that the cross-section of the steering rail 30 can be the union of rectangles intersecting at right (or another) angle, such as a plus-sign. Figure 12e illustrates that the cross-section of the steering rail 30 can be hexagonal. Figure 12f illustrates that the cross-section of the steering rail 30 can be rectangular or square with sharp or rounded (chamfered) edges. The cross-section of the steering rail 30 can be triangular, pentagonal, heptagonal, or octagonal. The steering rail 30, whether internal or external to the device 14, can deliver torque around the longitudinal and/or transverse axes of the device. The steering rail 30 can have various cross sections at various lengths along the rail 30. The steering rail 30 can guide, pitch, yaw and roll the device 14 into a desired orientation or indication. The device 14 can be delivered with one or more internal and/or external rails 30 and/or a sheath 28 or neither.

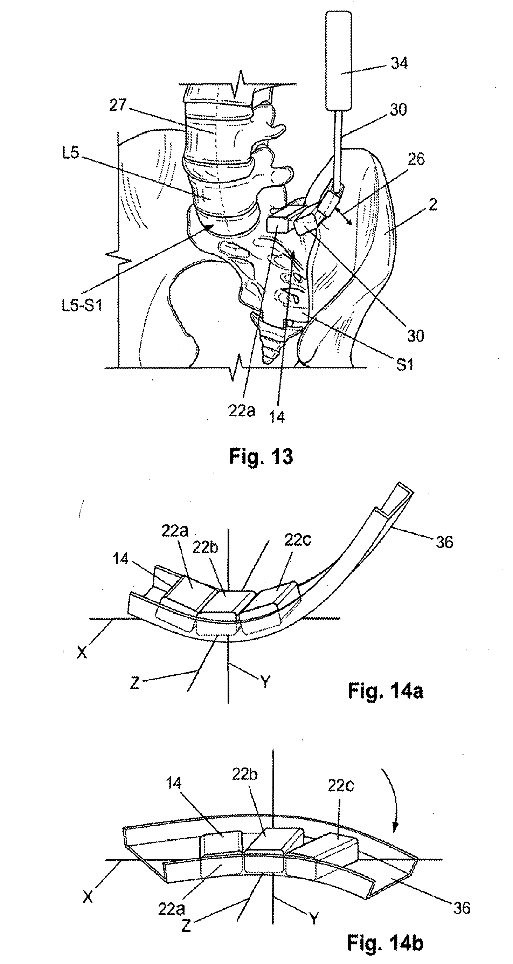

Figure 13 illustrates a device 14 that can be attached to a deployment tool having a controller handle 34 controllably attached to the internal steering rail 30. The internal steering rail 30 can pass through the device 14. The steering rail 30 can be fixedly attached to the device 14 during the delivery and articulation of the device 14. The device can be steered along or tracking the medial surface of the Ilium 2. The device 14 can then be positioned adjacent to the target site (e.g., the L5-S1 intervertebral space). The deployment tool can then release the device 14 from the steering rail 30 and push the device 14 into the target site.

Figures 14a and 14b illustrate that the device 14 can be delivered by being pushed along a steering horn, boot, or slide 36. The slide 36 can be similar to the steering tube 28, except that at least one wall of the slide 36 can be missing or open (e.g., the top wall is not present in the variation of the slide shown) compared with the steering tube 28. The missing wall can be completely open or replaced by one or more steering rails 30. The slide 36 can be used similar to the steering rails 30 and/or steering tube 28. The slide 36 can be steered, flexed or articulated by applying a tensile force to tensile cables within the rails, as shown by the arrow in Figure 14b, and the flexing from Figure 14a to 14b.

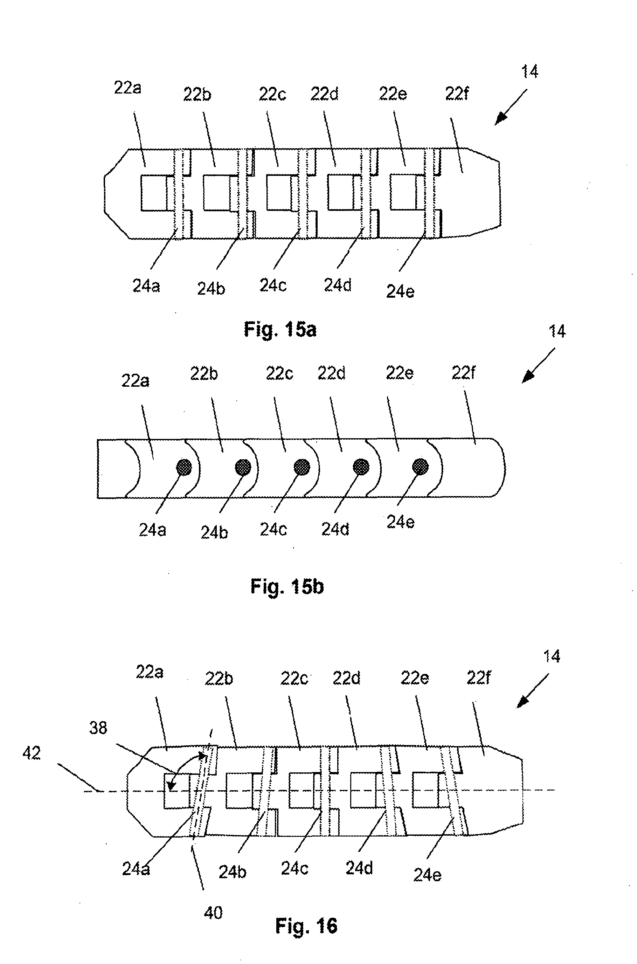

Figures 15a and 15b illustrate that the device 14 can have six segments 22a through 22f and five hinges 24a through 24e. The segments 22 can be attached to adjacent segments 22 by one or more hinges, tension or steering rails or wires, screws, pins, or combinations thereof. The hinges 24 can be pins. The segments 22 can be chained together. The segments 22 can be identical to each other except for the distal-most segment 22a and the proximal-most segment 22f. The segments 22 or links can be box-shaped. The hinges 24, such as the pins, can be parallel to all or some of the other hinges 24.

Figure 16 illustrates that the hinges 24 can be at acute angles to all or some of the hinges 24. The hinges 24 can be at hinge angles 38 with respect to each other. The hinge angle 38 can be measured between the hinge longitudinal axis 40 and the device longitudinal axis 42. The hinge angles 38 can be from about 80° to about 150°, more narrowly from about 90° to about 135°, yet more narrowly from about 95° to about 110°.

The device 14 can be translated and/or rotated by a handle 34 that can be removably attached to the device 14. The handle 34 can be screwed and/or snapped directly into the proximal end of the device 14, such as into the proximal-most segment 22. The handle 34 can compress, such as by grabbing or pinching, the proximal end of the device 14. The handle 34 can be a pusher, plunger, ram, or combinations thereof. The handle 34 and/or remainder of the deployment tool can be rigid and/or flexible or articulatable. For example, hinged similar to the device 14.

The segments 22 are not necessarily connected to each other by hinges. The segments 22 can be delivered to the target site individually, or as an unattached line of segments 22.

The device 14 can be cylindrical and flexible. The implantable device 14 can be fully flexible all the time. The device 14 can be mechanically stabilized by the deployment tool, steering wires, sheaths, tubes and guides. For example, the tools, wires, sheaths, tubes and guides can provide column stability to press the device 14 into the target site (e.g., intervertebral disc space).

The device 14 can flexible, and then locked with a tension or steering wire to stop rotational motion of the hinges once the device is delivered to and oriented within the target site. The tension wire could be tightened, for example by being tensioned by a nut to create higher friction in each hinge 24.

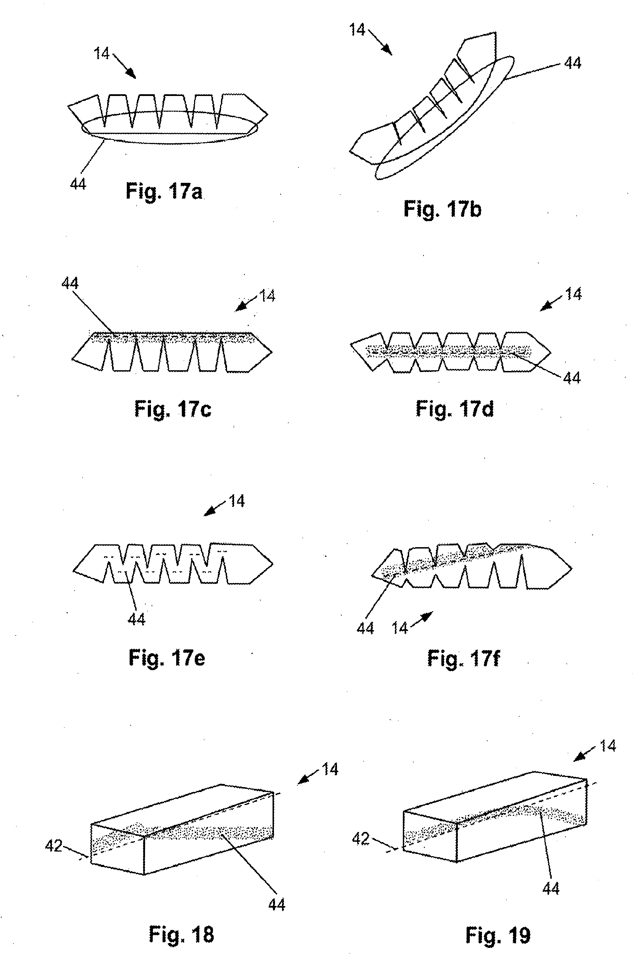

Figures 17a through 17f illustrate that the device 14 can have a living hinge 44. The living hinge 44 is a length of decreased rigidity and increased flexing within the body of the device 14. The living hinge 44 can be formed around slots and continuous segments of otherwise tough, durable material. The living hinge 44 can be defined be narrowing or thinning in the body of the device 14, such that the narrowing is sufficient to provide flexibility under reasonable torque. For example, the thickness of the unitary body of the device 14 at the living hinge 44 can be narrowed by more than about 85%, or more than about 90%, or more than about 95%, or more than about 97%, or more than about 98.5%. The living hinge 44 can have one or more repeated thinnings along the length of the device 14, as shown in Figures 17a through 17f.

Figures 17a and 17b illustrate that the device 14 bends at the living hinge 44. The living hinges 44 can be made to control the bend and direction of the device 14. The outer surface of the device 14 along the living hinge 44 can be smooth, for example providing low-friction surface for sliding over bone.

Figures 17a and 17b illustrate that the living hinge 44 can be along the bottom of the implant device 14. Figure 17c illustrates that the living hinge 44 can be along the top of the device 14. Figure 17d illustrates that the living hinge 44 can be through the middle or central axis of the device 14. Figure 17e illustrates that the living hinge 44 is discontinuous and on opposite sides of the center of the device 44. Figure 17f illustrates that the living hinge 44 is at an angle with respect to the longitudinal axis of the device 14, starting near the bottom of the device 14 and ending near the top of the device 14.

Figure 18 illustrates that the living hinge 42 can be at a non-zero angle to the central longitudinal axis 42 of the device 14. A first length of the living hinge 42 can be at a non-zero angle to a second length of the living hinge 44.

Figure 19 illustrates that the living hinge 44 can be curved. The living hinge 44 can curve around the central longitudinal axis 42 of the device 14.

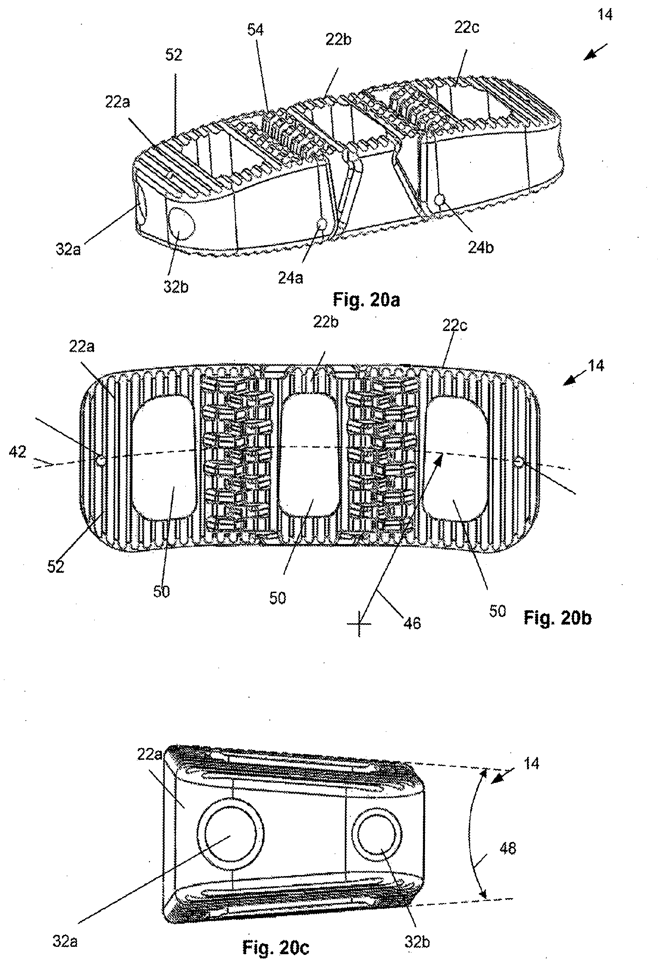

Figures 20a through 20c illustrate that the device can have three segments 22a, 22b and 22c connected by two hinges 24a and 24b. The device longitudinal axis 42 can be straight or can have a longitudinal radius of curvature 46. The longitudinal radius of curvature 46 can be from about 3 cm to about 100 cm, more narrowly from about 5 cm to about 20 cm, yet more narrowly from about 7 cm to about 15 cm, for example about 15 cm, also for example about 10 cm.

The device 14 can have an anterior taper angle 48. The taper angle can be measured between the plane of the top surface and the plane of the bottom surface of the device 14. The taper angle can be from about 0° (i.e., parallel top and bottom planes) to about 45°, more narrowly from about 2° to about 20°, yet more narrowly from about 4° to about 10°.

One or more segments have through-ports 50. The through-ports 50 can extend partially or completely form the top to the bottom surface of the device 14. The through-ports can be filled with a matrix or material to promote bone ingrowth; such as BMP or other materials listed herein.

The device 14 can have a surface coating or texturing on the top, and/or bottom, and/or side surfaces, such as lateral teeth 52, longitudinal or angled teeth, knurling, a coating or matrix to promote bone ingrowth, or combinations thereof.

The device 14 can have hinge teeth 54. The hinge teeth 54 can slide by adjacent hinge teeth to increase lateral stability during articulation and increase range of motion (e.g., a hinge tooth 54 on one segment 22 can slide into the gap between hinge teeth 54 on the adjacent segment 22 during articulation of the device 14).

One or more tension and/or steering wires can be inserted and/or tensioned through guide ports or channels 32a and 32b. The guide channels 32a and 32b can extend longitudinally through some or all of the segments 22.

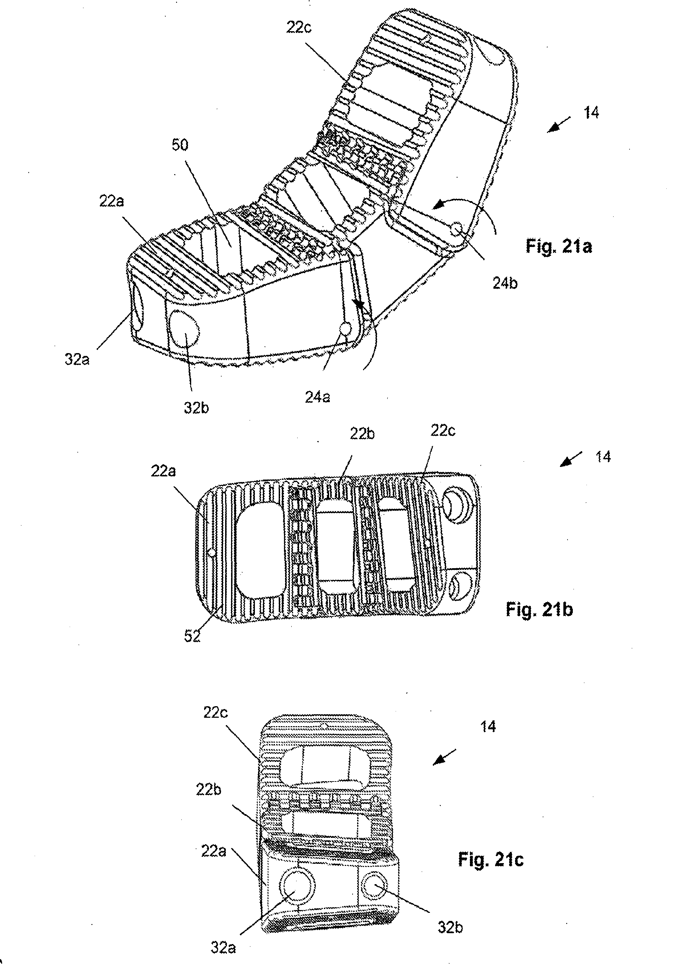

Figures 21a through 21c illustrate that device 14 can articulate. The segments 22 can rotate with respect to each other about the hinges 24, as shown by arrows.

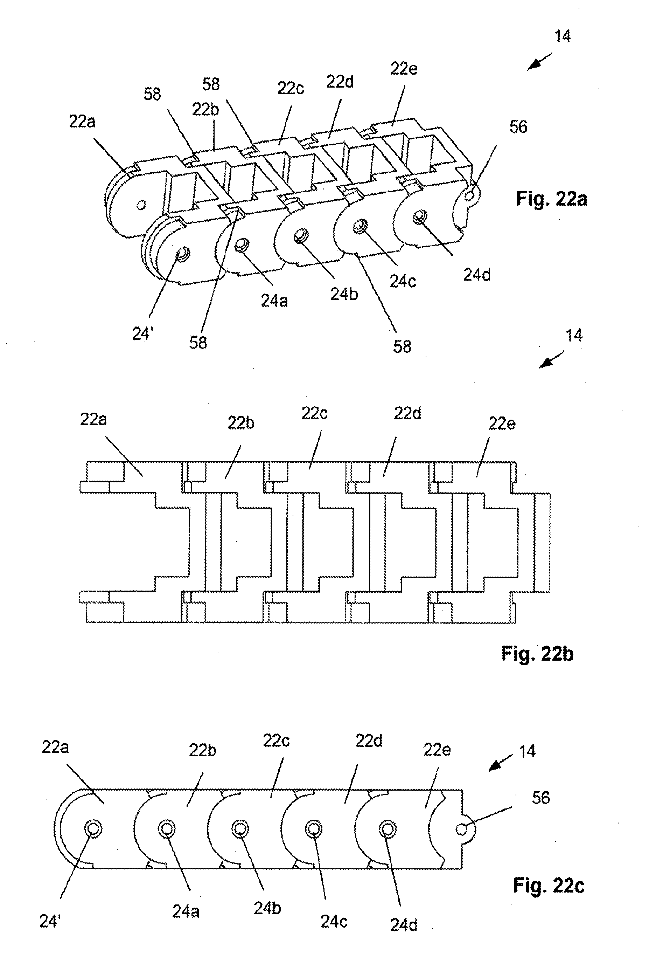

Figures 22a through 22c illustrate that some or all of the distal-most segments 22a through 22d can be identical. Segments 22 can be added or removed from the device 14, before during or after deployment to the target site, to increase or decrease the length of the device 14 to best fit the target site. The false hinge 24' can be a hinge component that is not attached to the other half of the hinge 24. The hinges 24 can snap together and apart. The articulation of each segment 22 can be limited by the interference fit of a rotational stop 58 on the top and bottom of the adjacent segment 22.

The device 14 can have a deployment tool interface, such as the lateral hole 56, for attaching to the deployment tool.

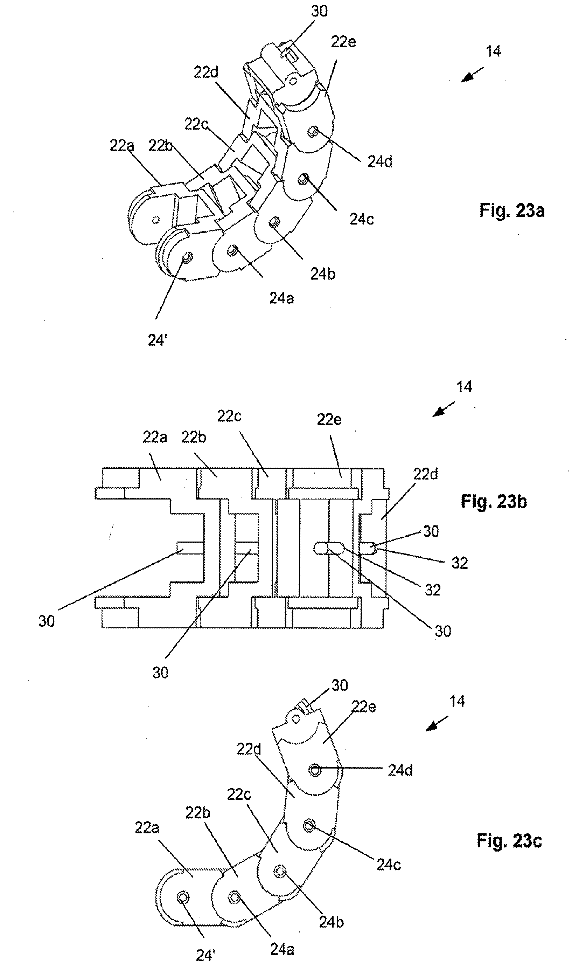

Figures 23a through 23c illustrate that a tensioning or steering wire or rail 30 can be deployed through the channels 32 on each segment. The wire 30 can then be tensioned to articulate and/or lock the device 14 in an articulated configuration.

Any or all elements of the device and/or other devices or apparatuses described herein can be made from, for example, a single or multiple stainless steel alloys, nickel titanium alloys (e.g., Nitinol), cobalt-chrome alloys (e.g., ELGILOY® from Elgin Specialty Metals, Elgin, IL; CONICHROME® from Carpenter Metals Corp., Wyomissing, PA), nickel-cobalt alloys (e.g., MP35N® from Magellan Industrial Trading Company, Inc., Westport, CT), molybdenum alloys (e.g., molybdenum TZM alloy, for example as disclosed in International Pub. No. WO 03/082363 A2, published 9 October 2003), tungsten-rhenium alloys, for example, as disclosed in International Pub. No. WO 03/082363, polymers such as polyethylene teraphathalate (PET)/polyester (e.g., DACRON® from E. I. Du Pont de Nemours and Company, Wilmington, DE), polypropylene, (PET), polytetrafluoroethylene (PTFE), expanded PTFE (ePTFE), polyether ketone (PEK), polyether ether ketone (PEEK), poly ether ketone ketone (PEKK) (also poly aryl ether ketone ketone), nylon, polyether-block co-polyamide polymers (e.g., PEBAX® from ATOFINA, Paris, France), aliphatic polyether polyurethanes (e.g., TECOFLEX® from Thermedics Polymer Products, Wilmington, MA), polyvinyl chloride (PVC), polyurethane, thermoplastic, fluorinated ethylene propylene (FEP), absorbable or resorbable polymers such as polyglycolic acid (PGA), polylactic acid (PLA), polycaprolactone (PCL), polyethyl acrylate (PEA), polydioxanone (PDS), and pseudo-polyamino tyrosine-based acids, extruded collagen, silicone, zinc, echogenic, radioactive, radiopaque materials, a biomaterial (e.g., cadaver tissue, collagen, allograft, autograft, xenograft, bone cement, morselized bone, osteogenic powder, beads of bone) any of the other materials listed herein or combinations thereof. Examples of radiopaque materials are barium sulfate, zinc oxide, titanium, stainless steel, nickel-titanium alloys, tantalum and gold.

Any or all elements of the device and/or other devices or apparatuses described herein, can be, have, and/or be completely or partially coated with agents and/or a matrix a matrix for cell ingrowth or used with a fabric, for example a covering (not shown) that acts as a matrix for cell ingrowth. The matrix and/or fabric can be, for example, polyester (e.g., DACRON® from E. I. Du Pont de Nemours and Company, Wilmington, DE), polypropylene, PTFE, ePTFE, nylon, extruded collagen, silicone or combinations thereof.

The device and/or elements of the device and/or other devices or apparatuses described herein and/or the fabric can be filled, coated, layered and/or otherwise made with and/or from cements, fillers, glues, and/or an agent delivery matrix known to one having ordinary skill in the art and/or a therapeutic and/or diagnostic agent. Any of these cements and/or fillers and/or glues can be osteogenic and osteoinductive growth factors.

Examples of such cements and/or fillers includes bone chips, demineralized bone matrix (DBM), calcium sulfate, coralline hydroxyapatite, biocoral, tricalcium phosphate, calcium phosphate, polymethyl methacrylate (PMMA), biodegradable ceramics, bioactive glasses, hyaluronic acid, lactoferrin, bone morphogenic proteins (BMPs) such as recombinant human bone morphogenetic proteins (rhBMPs), other materials described herein, or combinations thereof.

The agents within these matrices can include any agent disclosed herein or combinations thereof, including radioactive materials; radiopaque materials; cytogenic agents; cytotoxic agents; cytostatic agents; thrombogenic agents, for example polyurethane, cellulose acetate polymer mixed with bismuth trioxide, and ethylene vinyl alcohol; lubricious, hydrophilic materials; phosphor cholene; anti-inflammatory agents, for example non-steroidal antiinflammatories (NSAIDs) such as cyclooxygenase-1 (COX-1) inhibitors (e.g., acetylsalicylic acid, for example ASPIRIN® from Bayer AG, Leverkusen, Germany; ibuprofen, for example ADVIL® from Wyeth, Collegeville, PA; indomethacin; mefenamic acid), COX-2 inhibitors (e.g., VIOXX® from Merck & Co., Inc., Whitehouse Station, NJ; CELEBREX® from Pharmacia Corp., Peapack, NJ; COX-1 inhibitors); immunosuppressive agents, for example Sirolimus (RAPAMUNE®, from Wyeth, Collegeville, PA), or matrix metalloproteinase (MMP) inhibitors (e.g., tetracycline and tetracycline derivatives) that act early within the pathways of an inflammatory response. Examples of other agents are provided in Walton et al, Inhibition of Prostoglandin E2 Synthesis in Abdominal Aortic Aneurysms, Circulation, July 6, 1999, 48-54; Tambiah et al, Provocation of Experimental Aortic Inflammation Mediators and Chlamydia Pneumoniae, Brit. J. Surgery 88 (7), 935-940; Franklin et al, Uptake of Tetracycline by Aortic Aneurysm Wall and Its Effect on Inflammation and Proteolysis, Brit. J. Surgery 86 (6), 771-775; Xu et al, Sp1 Increases Expression of Cyclooxygenase-2 in Hypoxic Vascular Endothelium, J. Biological Chemistry 275 (32) 24583-24589; and Pyo et al, Targeted Gene Disruption of Matrix Metalloproteinase-9 (Gelatinase B) Suppresses Development of Experimental Abdominal Aortic Aneurysms, J. Clinical Investigation 105 (11), 1641-1649.

Any elements described herein as singular can be pluralized (i.e., anything described as "one" can be more than one). Any species element of a genus element can have the characteristics or elements of any other species element of that genus. The above-described configurations, elements or complete assemblies and methods and their elements for carrying out the invention, and variations of aspects of the invention can be combined and modified with each other in any combination.

## Claims

1. A biological implant device for providing orthopedic support, wherein the device has a device longitudinal axis, the device comprising:
a first rigid section (22a) with a first top plate and a first bottom plate; and
a second rigid section (22b) with a second top plate and a second bottom plate;
wherein a first longitudinal end of the first rigid section (22a) is rotatably attached to a second longitudinal end of the second rigid section (22b), and wherein the top and bottom plates are configured to interface with hard tissue,
**characterized in that**
the first rigid section (22a) is removable from the second rigid section (22b).

2. The device of Claim 1, wherein the device is configured such that in a first configuration the longitudinal axis of the device is straight, and wherein in a second configuration the longitudinal axis of the device has a radius of curvature of less than about 100 cm.

3. The device of Claim 1, further comprising an elongated element (30), and wherein the elongated element (30) extends laterally through the first rigid section (22a) and the second rigid section (22b), and wherein at least one of the first rigid section (22a) and second rigid section (22b) are rotatable around the elongated element (30).

4. The device of Claim 3, wherein the elongated element (30) comprises a pin.

5. The device of Claim 1, further comprising a tensioning element extending through the first rigid element (22a) and the second rigid element (22b).

6. The device of Claim 1, wherein the device has a taper angle (48), and wherein the taper angle (48) is greater than 4°.

7. The device of Claim 1, further comprising a third rigid section (22c), wherein the third rigid section comprises a third top plate and a third bottom plate, and wherein a second longitudinal end of the third rigid section (22c) is rotatably attached to a first longitudinal end of the second rigid section (22b).

8. The device of Claim 7, wherein the first longitudinal end of the second rigid section (22b) is longitudinally opposite to the second longitudinal end of the second rigid section (22b).

9. The device of Claim 1, wherein the first rigid section (22a) has a first longitudinal channel (32) extending through the first rigid section (22a), and wherein the second rigid section (22b) has a second longitudinal channel extending through the second rigid section.

10. The device of Claim 9, further comprising a tensioning element extending through the first longitudinal channel (32) and the second longitudinal channel (32).

11. The device of Claim 1, further comprising an external steering element (28; 30a; 30b).

12. The device of Claim 11, wherein the external steering element (30a, 30b) comprises a rail extending longitudinally along the lateral side of the first rigid section (22a) and the second rigid section (22b).

13. The device of Claim 11, wherein the external steering element (28) comprises at least one of a steering tube or slide.

14. The device of Claim 1, further comprising an internal steering element (30).

## Patentansprüche

1. Biologische Implantatvorrichtung zur Bereitstellung einer orthopädischen Unterstützung, wobei die Vorrichtung eine Vorrichtungslängsachse besitzt und Folgendes umfasst:
einen ersten starren Abschnitt (22a) mit einer ersten Deckplatte und einer ersten Bodenplatte; und
einen zweiten starren Abschnitt (22b) mit einer zweiten Deckplatte und einer zweiten Bodenplatte;
wobei ein erstes Längsende des ersten starren Abschnitts (22a) drehbar an einem zweiten Längsende des zweiten starren Abschnitts (22b) befestigt ist und wobei die Deck- und Bodenplatten so gestaltet sind, dass sie an Hartgewebe angrenzen,
**dadurch gekennzeichnet, dass**
der erste starre Abschnitt (22a) von dem zweiten starren Abschnitt (22b) abnehmbar ist.

2. Vorrichtung nach Anspruch 1, wobei die Vorrichtung so gestaltet ist, dass in einer ersten Ausführung die Längsachse der Vorrichtung gerade ist, und wobei die Längsachse der Vorrichtung in einer zweiten Ausführung einen Krümmungsradius von weniger als etwa 100 cm hat.

3. Vorrichtung nach Anspruch 1, ferner umfassend ein langgestrecktes Element (30), wobei sich das langgestreckte Element (30) seitlich durch den ersten starren Abschnitt (22a) und den zweiten starren Abschnitt (22b) erstreckt, und wobei mindestens einer von dem ersten starren Abschnitt (22a) und dem zweiten starren Abschnitt (22b) um das langgestreckte Element (30) drehbar ist.

4. Vorrichtung nach Anspruch 3, wobei das langgestreckte Element (30) einen Stift umfasst.

5. Vorrichtung nach Anspruch 1, ferner umfassend ein Spannelement, das sich durch das erste starre Element (22a) und das zweite starre Element (22b) erstreckt.

6. Vorrichtung nach Anspruch 1, wobei die Vorrichtung einen Kegelwinkel (48) besitzt und wobei der Kegelwinkel (48) größer ist als 4°.

7. Vorrichtung nach Anspruch 1, ferner umfassend einen dritten starren Abschnitt (22c), wobei der dritte starre Abschnitt eine dritte Deckplatte und eine dritte Bodenplatte umfasst und wobei ein zweites Längsende des dritten starren Abschnitts (22c) drehbar an einem ersten Längsende des zweiten starren Abschnitts (22b) befestigt ist.

8. Vorrichtung nach Anspruch 7, wobei das erste Längsende des zweiten starren Abschnitts (22b) dem zweiten Längsende des zweiten starren Abschnitts (22b) in Längsrichtung entgegengesetzt ist.

9. Vorrichtung nach Anspruch 1, wobei der erste starre Abschnitt (22a) einen sich durch den ersten starren Abschnitt (22a) erstreckenden ersten Längskanal (32) besitzt, und wobei der zweite starre Abschnitt (22b) einen sich durch den zweiten starren Abschnitt erstreckenden zweiten Längskanal besitzt.

10. Vorrichtung nach Anspruch 9, ferner umfassend ein Spannelement, das sich durch den ersten Längskanal (32) und den zweiten Längskanal (32) erstreckt.

11. Vorrichtung nach Anspruch 1, ferner umfassend ein externes Lenkelement (28; 30a; 30b).

12. Vorrichtung nach Anspruch 11, wobei das externe Lenkelement (30a, 30b) eine Schiene umfasst, die sich in Längsrichtung entlang der lateralen Seite des ersten starren Abschnitts (22a) und des zweiten starren Abschnitts (22b) erstreckt.

13. Vorrichtung nach Anspruch 11, wobei das externe Lenkelement (28) mindestens eines von einem Lenkrohr oder einem Schieber umfasst.

14. Vorrichtung nach Anspruch 1, ferner umfassend ein internes Lenkelement (30).

## Revendications

1. Dispositif pour implant biologique, destiné à procurer un support orthopédique, dans lequel le dispositif présente un axe longitudinal de dispositif, le dispositif comprenant :
une première section rigide (22a) dotée d'une première plaque supérieure et d'une première plaque inférieure ; et
une seconde section rigide (22b) dotée d'une seconde plaque supérieure et d'une seconde plaque inférieure ;
dans lequel une première extrémité longitudinale de la première section rigide (22a) est fixée de manière à pouvoir tourner à une seconde extrémité longitudinale de la section rigide (22b), et dans lequel les plaques supérieure et inférieure sont configurées pour faire interface avec un tissu dur,
**caractérisé en ce que** :
la première section rigide (22a) est détachable de la seconde section rigide (22b).

2. Dispositif selon la revendication 1, dans lequel le dispositif est configuré de manière que dans une première configuration, l'axe longitudinal du dispositif soit droit, et dans lequel, dans une seconde configuration, l'axe longitudinal du dispositif présente un rayon de courbure inférieur à environ 100 cm.

3. Dispositif selon la revendication 1, comprenant en outre un élément allongé (30), et dans lequel l'élément allongé (30) s'étend latéralement à travers la première section rigide (22a) et la seconde section rigide (22b) et dans lequel au moins l'une de la première section rigide (22a) et de la seconde section rigide (22b) peut tourner autour de l'élément allongé (30).

4. Dispositif selon la revendication 3, dans lequel l'élément allongé (30) comprend une broche.

5. Dispositif selon la revendication 1, comprenant en outre un élément tendeur s'étendant à travers le premier élément rigide (22a) et le second élément rigide (22b).

6. Dispositif selon la revendication 1, dans lequel le dispositif présente un angle de conicité (48) et dans lequel l'angle de conicité (48) est supérieur à 4°.

7. Dispositif selon la revendication 1, comprenant en outre une troisième section rigide (22c), dans lequel la troisième section rigide comprend une troisième plaque supérieure et une troisième plaque inférieure, et dans lequel une seconde extrémité longitudinale de la troisième section rigide (22c) est fixée de manière à pouvoir tourner à une première extrémité longitudinale de la deuxième section rigide (22b).

8. Dispositif selon la revendication 7, dans lequel la première extrémité longitudinale de la deuxième section rigide (22b) est longitudinalement opposée à la seconde extrémité longitudinale de la deuxième section rigide (22b).

9. Dispositif selon la revendication 1, dans lequel la première section rigide (22a) présente un premier canal longitudinal (32) s'étendant à travers la première section rigide (22a), et dans lequel la seconde section rigide (22b) présente un second canal longitudinal s'étendant à travers la seconde section rigide.

10. Dispositif selon la revendication 9, comprenant en outre un élément tendeur s'étendant à travers le premier canal longitudinal (32) et le second canal longitudinal (32).

11. Dispositif selon la revendication 1, comprenant en outre un élément externe de direction (28 ; 30a ; 30b).

12. Dispositif selon la revendication 11, dans lequel l'élément externe de direction (30a, 30b) comprend un rail s'étendant longitudinalement le long du côté latéral de la première section rigide (22a) et de la seconde section rigide (22b).

13. Dispositif selon la revendication 11, dans lequel l'élément externe de direction (28) comprend au moins un tube de direction ou une glissière.

14. Dispositif selon la revendication 1, comprenant en outre un élément interne de direction (30).
